# EUROPEAN PATENT SPECIFICATION

(11) **EP 3 722 807 B1**
(45) Date of publication and mention of the grant of the patent: **15.02.2023**
(21) Application number: 19168889.4
(22) Date of filing: 12.04.2019
(51) Int. Cl.: G01N 33/483, B01L 3/00, C12M 3/06, G01N 33/50

(54) **MICROFLUIDIC DEVICE FOR MEASURING CELL IMPEDANCE AND TRANSEPITHELIAL ELECTRICAL RESISTANCE**
MIKROFLUIDISCHE VORRICHTUNG ZUR MESSUNG VON ZELLIMPEDANZ UND TRANSEPITHELIALEM ELEKTRISCHEM WIDERSTAND
DISPOSITIF MICROFLUIDIQUE DE MESURE DE L'IMPÉDANCE DE CELLULES ET DE LA RÉSISTANCE ÉLECTRIQUE TRANSEPITHELIALE

(43) Date of publication of application: 14.10.2020
(73) Proprietor: Technische Universität Wien, 1040 Wien (AT)
(72) Inventor: Schuller, Patrick, 1130 Wien (AT); Rothbauer, Mario, 2511 Pfaffstätten (AT); Wanzenböck, Heinz, 1160 Wien (AT); Ertl, Peter, 1190 Wien (AT)
(74) Representative: Pföstl, Andreas

(56) References cited:
- US-A1- 2014 065 660
- TOMMASO SBRANA ET AL: "Dual flow bioreactor with ultrathin microporous TEER sensing membrane for evaluation of nanoparticle toxicity", SENSORS AND ACTUATORS B: CHEMICAL, vol. 223, 16 September 2015 (2015-09-16), pages 440-446, XP055658655, ISSN: 0925-4005, DOI: 10.1016/j.snb.2015.09.078

## Description

### TECHNICAL FIELD

The present invention relates to the field of microfluidic devices and the measurement of cell impedance and transepithelial electrical resistance (TEER) of cells and cell layers.

### BACKGROUND ART

Monolayers of epithelial and endothelial cells form barriers within animals, in particular within humans, and regulate the free movement of molecules between different tissues and/or interstitial compartments. In many diseases, these barriers become compromised, and hence, measuring their permeability is of considerable interest to cell biologists.

Most epithelial and endothelial cells types can be cultured in vitro to form confluent monolayers where it is possible to measure the barrier function afforded by these cell layers. In addition, dynamic changes of the layers can be followed when the cellular environment is altered by exposure to substances (e.g. pharmaceutical compounds, toxic compounds) or physical changes such as mechanical or osmotic stress.

The barrier function (permeability) of cell monolayers can be measured using various methods. Some of these methods are based on the measurement of electrical resistance or impedance. The cells and cell layer to be examined is usually grown upon a solid substrate, upon a membrane filter or on a porous membrane.

Cells can be monitored upon a solid substrate with gold electrodes and typically performed using 96 well arrays. Alternatively, a cell layer may be applied on a membrane filter or a porous membrane as a substrate. The use of the aforementioned filters and membranes is advantageous since it simulates a more in vivo like situation where cells are effectively fed from both the apical and basal side. It is commonly observed that under these conditions cell layers achieve higher absolute barrier function.

Sbrana T. et al (Sensors and Actuators B: Chemical 223 (2015): 440-446) discloses the design and fabrication of a transepithelial electrical resistance (TEER)-bioreactor for the study of nanoparticle toxicity in intestinal epithelial cells.

US 2014/065660 A1 discloses a microfluidic biological barrier model, e.g. for measuring the transepithelial electrical resistance (TEER).

The measurement of electrical resistance and impedance requires the presence of electrodes close to the cells and cell layer. Current methods do not allow the measurement of transepithelial electrical resistance (TEER) and/or for determining the impedance in an accurate simulating an in vivo like situation where the cells are located on a porous membrane because the cells cannot be positioned close to the electrodes. Hence, it is an object of the present invention to provide methods and means allowing to determine the transepithelial electrical resistance (TEER) of a cell layer and/or the impedance of cells or a cell layer using porous membranes.

### SUMMARY OF THE INVENTION

The present invention relates to a microfluidic device for determining the transepithelial electrical resistance (TEER) of a cell layer or a cell assembly and/or for determining the impedance of cells, a cell layer or a cell assembly, said device comprising at least one microchannel comprising at least a lower and an upper compartment separated by at least two porous membranes and at least one inner compartment, the lower compartment comprising a bottom wall and side walls, the upper compartment comprising an upper wall and side walls, the bottom and upper wall, the side walls and the at least two porous membranes defining compartment volumes, wherein at least one porous membrane comprises on its surface at least one electrode, wherein the at least two porous membranes and side walls define the at least one inner compartment volume being positioned between the lower and the upper compartment.

It turned surprisingly out that with the method of the present invention for producing a porous membrane comprising an electrode on its surface it is possible to apply electrodes in any design (e.g. interdigitated, strip or disc electrode) on porous and flexible membranes. This allows to manufacture microfluidic devices as defined above comprising electrodes on such porous and flexible membranes which are not supported by a stiff or inflexible solid support. The presence of electrodes on porous membranes makes it possible to determine the transepithelial electrical resistance (TEER) of a cell layer or a cell assembly, preferably hydrogel-free or hydrogel-containing three-dimensional cell assemblies, directly and more precisely because the electrodes on the porous membrane can be positioned directly in the vicinity of or beneath a cell layer.

A further advantage of the microfluidic device of the present invention is the possibility to perform TEER measurements without the influence of a porous membrane since the electrodes are positioned on a porous membrane in direct contact with a cell layer or cell assembly. Furthermore, the microfluidic device of the present invention allows to measure the cumulative TEER of cell layers and hydrogel-free or hydrogel-containing three-dimensional cell assemblys positioned on both sides of a porous membrane. Devices and methods known in the art require to perform two independent measurements.

Hence, another aspect of the present invention relates to a method for determining the transepithelial electrical resistance (TEER) of a cell layer comprising the steps of
- providing a device according to the present invention comprising at least one electrode on the surface of at least one porous membrane facing the upper and/or lower compartment and/or an inner compartment and at least one electrode on the surface of the upper wall and/or side wall of the upper compartment and/or the bottom wall and/or side wall of the lower compartment, wherein the porous membrane is covered by a cell layer or cell assembly,
- applying a direct current to
   - at least one electrode on said porous membrane facing the upper compartment or inner compartment and to at least one electrode on the surface of the upper wall and/or side wall of the upper compartment if the porous membrane facing the upper compartment is covered by a cell layer or cell assembly, or
   - at least one electrode on said porous membrane facing the lower compartment or inner compartment and to at least one electrode on the surface of the upper wall and/or side wall of the upper compartment if the porous membrane facing the upper compartment is covered by a cell layer or cell assembly, or
   - at least one electrode on said porous membrane facing the lower compartment or inner compartment and to at least one electrode on the surface of the bottom wall and/or side wall of the lower compartment if the porous membrane facing the lower compartment is covered by a cell layer or cell assembly, or
   - at least one electrode on said porous membrane facing the upper compartment or inner compartment and to at least one electrode on the surface of the bottom wall and/or side wall of the lower compartment if the porous membrane facing the lower compartment is covered by a cell layer or cell assembly, or
   - at least one electrode on the surface of the upper wall and/or side wall of the upper compartment and to at least one electrode on the surface of the bottom wall and/or side wall of the lower compartment if the porous membrane facing the lower and/or compartment is covered by a cell layer or cell assembly, or
   - at least one electrode on a first porous membrane and to at least one electrode on a second porous membrane defining, the first and the second porous membrane defining the inner compartment, wherein the inner compartment comprises a cell assembly or a cell layer covering the first and/or second porous membrane,,
      and
- measuring the electrical resistance.

The device of the present invention can also be used to determine the impedance of cells or cell layers by applying alternating current to the device comprising cells and/or cell layers being present on the porous membrane and/or any wall of the compartments of the microfluidic channel.

Thus, a further aspect of the present invention relates to a method for determining the impedance of cells, a cell layer or cell assembly comprising the steps of
- providing a device according to the present invention comprising at least one electrode on the surface of at least one porous membrane facing the upper and/or lower compartment and/or an inner compartment and optionally at least one electrode on the surface of the upper wall and/or side wall of the upper compartment and/or the bottom wall and/or side wall of the lower compartment, wherein the porous membrane is covered by cells, a cell layer or a cell assembly,
- applying an alternating current to
   - at least two electrodes on said porous membrane being covered by said cells, cell layer or cell assembly, or
   - at least one electrode on said porous membrane facing the upper compartment and to at least one electrode on the surface of the upper wall and/or side wall of the upper compartment if the porous membrane facing the upper compartment is covered by said cells, cell layer or cell assembly, or
   - at least one electrode on said porous membrane facing the lower compartment and to at least one electrode on the surface of the upper wall and/or side wall of the upper compartment if the porous membrane facing the upper compartment is covered by said cells, cell layer or cell assembly, or
   - at least one electrode on said porous membrane facing the lower compartment and to at least one electrode on the surface of the bottom wall and/or side wall of the lower compartment if the porous membrane facing the lower compartment is covered by said cells, cell layer or cell assembly, or
   - at least one electrode on said porous membrane facing the upper compartment and to at least one electrode on the surface of the bottom wall and/or side wall of the lower compartment if the porous membrane facing the lower compartment is covered by said cells, cell layer or cell assembly, or
   - at least one electrode on the surface of the upper wall and/or side wall of the upper compartment and to at least one electrode on the surface of the bottom wall and/or side wall of the lower compartment if the porous membrane facing the lower and/or compartment is covered by said cells, cell layer or cell assembly,
      and
- measuring the impedance or capacitance.

The provision of electrodes on porous membranes, in particular on flexible porous membranes, as used in the devices of the present invention cannot be achieved using methods in the art.

### BRIEF DESCRIPTION OF THE FIGURES

Figs. 1A-C show cross sections of microchannels being part of microfluidic devices, wherein Figs. 1B and 1C show embodiments of the present invention.
Fig. 2 shows interdigitated electrodes on a porous membrane
Fig. 3 shows a strip electrode on a porous membrane
Fig. 4 shows a disc electrode on a porous membrane
Figs. 5 to 16 show cross sections of various microchannel setups not within the scope of the present invention, showing cell layers positioned on the bottom and/or upper wall and/or on one or both surfaces of the porous membrane. These figures show combinations of cells grown on the microfluidic cell barrier chip as well as different combinations of the electrodes to allow for TEER measurements of the whole cell barrier (up to quadruple co-culture), single cell type barriers, media sensing and impedance spectroscopy of the cell barrier.

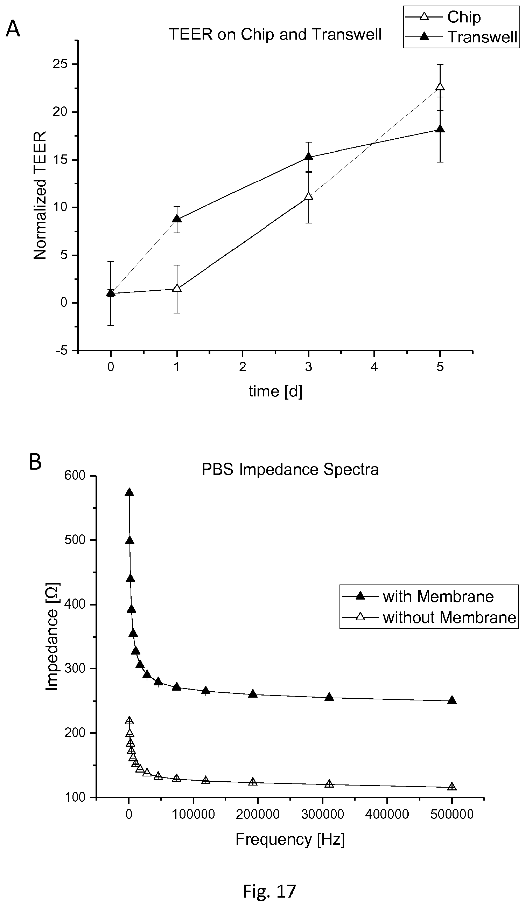

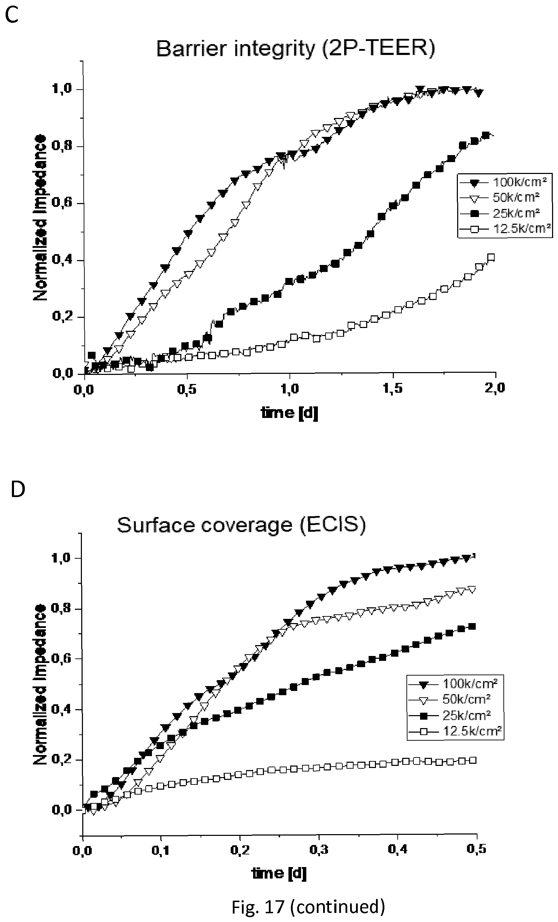

Fig. 17 shows that (A) the 2-point TEER measurement using 100 µm gap-to-finger interdigitated thin film electrodes result in a similar slope than trans-well TEER measurements using chopstick electrodes in trans-wells, (B) Using the membrane-bound electrodes true cellular resistance based on tight junctions can be measured in the absence of membrane resistance (no membrane TEER subtraction necessary, which is used to normalize conventional systems; prone to inaccuracies), and (C,D) membrane bound interdigitated electrodes of (C) 100 µm gap-to-finger ratio can be used for monitoring cellular resistance in parallel to 15 µm finger-to-gap interdigitated next to the 100 µm electrode to determine cell-surface coverage at the same time for multi-parametric evaluation of cell barrier parameters such as integrity (tight junctions) and coverage.

### DESCRIPTION OF EMBODIMENTS

The methods and devices described herein allow to study cell barriers using electrical impedance spectroscopy and/or TEER. The combination of thin film electrodes of any architecture located on porous, free-standing and flexible polymer membranes allows for the first time versatile interconnection of multiple electrodes in a microfluidic device of the present invention. This means that, compared to conventional TEER and impedance strategies, where just a single barrier plane can be tested, multiple cell barriers can be probed within a single multi-layered device using a variety of electrode designs including disc, band and interdigitated metal thin film electrodes. The microfluidic device of the present invention allows to correlate cell surface coverage using membrane-integrated metal thin film electrodes (2-point impedance measurement setup) with barrier integrity and tightness based on Trans-cellular resistance measurements (TEER; 4-point measurement setup).

The present invention in based on the combination of well-known electro-analytical biosensing strategies (electrical impedance spectroscopy and TEER) for cell and cell barriers analysis, thus creating synergetic effects that compensate for the individual limitations of both analytical methods. As a consequence, more complex biological structures comprising of multiple physiological cell layers can be investigated using a single method and a single microfluidic device.

As mentioned above the present invention allows for the first time to solve the problem of produce reliable structured metal films of a defined geometry (i.e. electrodes) down to 2.5 µm or less on porous, flexible polymeric membranes by using a simplified method that allows the fabrication of even highly interdigitated metal thin film electrodes on even 10 µm or less thick flexible porous and free-standing polymeric membranes.

The microfluidic device of the present invention may have any architecture provided that the device comprises at least one, preferably at least two, more preferably at least three, more preferably at least three, microchannels as defined herein. The at least one microchannel comprises three or more compartments, a lower and an upper compartment and at least one inner compartment. These at least three compartments may have different inlets and different outlets so that both compartments may not be fluidly connected to each other. However, it is possible that one or more microchannels of the microfluidic device of the present invention have the same inlets and/or outlets.

The device of the present invention can be used to determine the TEER of a cell layer or a cell assembly and/or for determining the impedance of cells, cell layer or a cell assembly. The cells may be eukaryotic cells, in particular mammalian cells. The cells may be epithelial or endothelial cells or stem cells capable to be differentiated into epithelial or endothelial cells.

The cell assembly used in the methods of the present invention may be a cell aggregate or any other combination of cells including hydrogel-based assemblies (e.g. cell mono- and co-cultures embedded in synthetic and/or natural hydrogels) as well as cellular self-assembly (e.g. spheroids). Hydrogels which can be used to embed cells include extracellular matrix extracts (Matrigel, Geltrex), fibrin hydrogels, silk hydrogels, collagen hydrogels, gelatin hydrogels, hydrogels from alginic acid (alginate) or composites thereof. Furthermore, synthetic hydrogels like dextran (e.g. PEG-dextran) can also be used.

The at least two porous membranes and side walls define the at least one inner compartment volume being positioned between the lower and upper compartment.

According to a further preferred embodiment of the present invention at least one electrode on at least one porous membrane is facing the lower and/or upper compartment and/or at least one inner compartment.

The porous membrane within the at least one microchannel of the device of the present invention may comprise at least one electrode on one or both sides of said membrane. However, it is preferred that only one side of the porous membrane comprises electrodes on its surface.

According to another preferred embodiment of the present invention the bottom and/or upper wall and/or at least one of the side walls of one or more compartments comprises at least one electrode on its surface, wherein it is particularly preferred that one or more compartments comprise on the bottom and/or upper wall (not the side walls) at least one electrode.

The porous membrane separating the compartments of the at least one microchannel comprise at least one electrode positioned on the surface of said porous membrane. However, also the upper wall, the bottom wall and/or the one or more sidewalls of the compartment may comprise at least one electrode positioned on their surface. This is particularly advantageous because it allows to determine the TEER of a cell layer by applying an electric current between the at least one electrode on the porous membrane covered by said cell layer and at least one electrode on the upper or bottom wall or side wall in a compartment resulting in the order electrode-cell layer-electrode.

According to a further preferred embodiment of the present invention the porous membrane and the bottom and/or upper wall and/or at least one of the side walls of one or more compartments comprise at least two electrodes on their surface.

The presence of at least two electrodes on the surface of the aforementioned elements of the at least one microchannel allows measuring impedance between the at least two electrodes whose electron flow can be impeded by the presence of cells or a cell layer on said surfaces. Furthermore, the provision of more than two electrodes on the surface of the porous membrane and the bottom and/or upper wall and/or at least one of the side walls of the one or more compartments increases the reliability of the TEER measurements.

According to a preferred embodiment of the present invention the at least one electrode on the surface of the at least one porous membrane is positioned substantially opposite to the at least one electrode on the surface a second porous membrane and/or of the lower and/or upper wall of one or more compartments.

It is advantageous to position the electrodes on the surface of the porous membrane substantially opposite to the at least one electrode on the surface of the lower first and/or upper wall of one or more compartments allowing a direct electron flow between at least two electrodes.

According to another preferred embodiment of the present invention the porous membrane comprises or consists of a polymer selected from the group consisting of polyesters, preferably polyethylene terephthalate, polystyrene, polycarbonate, polyether ether ketone (PEEK) or and thiol-ene polymers, preferably epoxy-containing thiol-ene polymers.

The aforementioned polymers can be used as part of or form themselves porous membranes which can be used in the device of the present invention.

The at least one porous membrane may have a thickness of 2 to 50 pm, preferably 5 to 20 pm, more preferably 5 to 15 pm, more preferably 8 to 12 pm, in particular approximately 10 µm.

According to a further embodiment of the present invention the side walls of the compartments of the at least one microchannel have a height of 1 to 1500 pm, preferably 5 to 1300 pm, more preferably 50 to 1250 pm, in particular approximately 1200 µm.

The electrodes comprise or consist preferably of a material selected from the group consisting of silver, gold, platinum, chromium, aluminium zinc oxide (AZO), indium tin oxide (ITO), iridium platinum, black platinum, titanium nitride and carbon.

The material used for manufacturing the electrodes on a surface within the microchannel of the device of the present invention should show a good electrical conductivity and be substantially inert against cell culture media and water used for cultivating cells or washing the microfluidic device of the present invention. Furthermore, the material shall be applicable on a surface using methods known in the art and shall be flexible enough to be resistant against movements of the porous membrane.

According to a preferred embodiment of the present invention the electrodes have a thickness of 10 to 1500 nm, preferably 15 to 250 nm, more preferably 50 to 75 nm, in particular approximately 70 nm.

According to a further preferred embodiment of the present invention at least two electrodes on said porous membrane, on said bottom wall, on said upper wall and on said side walls have a distance from each other ranging from 1 to 500 pm, preferably 10 to 250 pm, more preferably 10 to 100 pm, in particular approximately 15 µm.

The electrodes in the microfluidic device of the present invention may have different structures. Hence, according to preferred embodiment of the present invention the at least one porous membrane of the device of the present invention comprises on its surface at least two electrodes arranged as interdigitated electrodes having a plurality of digits forming a comb-like electrode pattern.

According to a preferred embodiment of the present invention
a) said at least one porous membrane comprises at least one electrode facing the upper compartment and/or at least one inner compartment and the upper wall and/or side wall of the upper compartment and/or the bottom wall and/or side wall of the lower compartment comprises at least one electrode on its surface , or
b) said at least one porous membrane comprises at least one electrode facing the lower compartment and/or at least one inner compartment and the upper wall and/or side wall of the upper compartment and/or the bottom wall and/or side wall of the lower compartment comprises at least one electrode on its surface , or c) said at least one porous membrane comprises at least one electrode facing the at least one inner compartment.

A device comprising such an arrangement can be used for determining the TEER of a cell layer or its impedance. In the course of a TEER measurement, the cell layer is preferably positioned between at least two electrodes wherein at least one electrode is positioned on the porous membrane and at least one electrode is positioned on the upper, bottom or a side wall of the compartment.

The surfaces of the microchannel, i.e. the porous membrane, the bottom wall, the upper wall and/or the side walls and optionally the electrodes, may be modified in a manner to prevent or support/allow the attachment of cells thereon. The provision of appropriate surface modifications allows to regulate the areas within the microchannel of the device of the present invention which are covered by cells or cell layers. In order to support the attachment of cells on a surface within the microchannel of the device of the present invention said surface may be modified with natural ECM proteins (collagen, fibronectin, vitronectin, laminin, ECM protein motifs (RGD motif containing molecules, hydrogels and/or self-assembly monolayers), silanes (e.g. APTES for amino groups) or synthetic hydrogel layers (e.g. RDG-modified PEG).

Another aspect of the present invention relates to a method for determining the transepithelial electrical resistance (TEER) of a cell layer or a cell assembly comprising the steps of
- providing a device according to the present invention comprising at least one electrode on the surface of a porous membrane facing the upper and/or lower compartment and at least one electrode on the surface of the upper wall and/or side wall of the upper compartment and/or the bottom wall and/or side wall of the lower compartment, wherein the porous membrane is covered by a cell layer or cell assembly,
- applying a direct current to
   - at least one electrode on said porous membrane facing the upper compartment or inner compartment and to at least one electrode on the surface of the upper wall and/or side wall of the upper compartment if the porous membrane facing the upper compartment is covered by a cell layer or cell assembly, or
   - at least one electrode on said porous membrane facing the lower compartment or inner compartment and to at least one electrode on the surface of the upper wall and/or side wall of the upper compartment if the porous membrane facing the upper compartment is covered by a cell layer or cell assembly, or
   - at least one electrode on said porous membrane facing the lower compartment or inner compartment and to at least one electrode on the surface of the bottom wall and/or side wall of the lower compartment if the porous membrane facing the lower compartment is covered by a cell layer or cell assembly, or
   - at least one electrode on said porous membrane facing the upper compartment or inner compartment and to at least one electrode on the surface of the bottom wall and/or side wall of the lower compartment if the porous membrane facing the lower compartment is covered by a cell layer or cell assembly, or
   - at least one electrode on the surface of the upper wall and/or side wall of the upper compartment and to at least one electrode on the surface of the bottom wall and/or side wall of the lower compartment if the porous membrane facing the lower and/or compartment is covered by a cell layer or cell assembly, or
   - at least one electrode on a first porous membrane and to at least one electrode on a second porous membrane defining, the first and the second porous membrane defining the inner compartment, wherein the inner compartment comprises a cell assembly or a cell layer covering the first and/or second porous membrane,
      and
- measuring the electrical resistance.

The microfluidic device of the present invention can be used for determining the transepithelial electrical resistance (TEER) of one or more cell layers. In order to determine TEER cells are applied to at least one microchannel of the device of the present invention. A cell layer shall be positioned preferably on the porous membrane comprising on its surface at least one electrode. The field strength applied during the measurements is typically less than typically applied for electroporating cells and determined empirically. Typically a voltage of 1 to 100 mV, preferably 2 to 90 mV, more preferably 5 to 80 mV, more preferably 10 to 70 mV, more preferably 30 to 60 mV, in particular 50 mV, is used.

A further aspect of the present invention relates to a method for determining the impedance of cells or a cell layer comprising the steps of
- providing a device according to the present invention comprising at least one electrode on the surface of at least one porous membrane facing the upper and/or lower compartment and/or an inner compartment and optionally at least one electrode on the surface of the upper wall and/or side wall of the upper compartment and/or the bottom wall and/or side wall of the lower compartment, wherein the porous membrane is covered by cells, a cell layer or a cell assembly, preferably a hydrogel-free cell assembly or a hydrogel-containing cell assembly,
- applying an alternating current to
   - at least two electrodes on said porous membrane being covered by said cells, cell layer or cell assembly, or
   - at least one electrode on said porous membrane facing the upper compartment and to at least one electrode on the surface of the upper wall and/or side wall of the upper compartment if the porous membrane facing the upper compartment is covered by said cells, cell layer or cell assembly, or
   - at least one electrode on said porous membrane facing the lower compartment and to at least one electrode on the surface of the upper wall and/or side wall of the upper compartment if the porous membrane facing the upper compartment is covered by said cells, cell layer or cell assembly, or
   - at least one electrode on said porous membrane facing the lower compartment and to at least one electrode on the surface of the bottom wall and/or side wall of the lower compartment if the porous membrane facing the lower compartment is covered by said cells, cell layer or cell assembly, or
   - at least one electrode on said porous membrane facing the upper compartment and to at least one electrode on the surface of the bottom wall and/or side wall of the lower compartment if the porous membrane facing the lower compartment is covered by said cells, cell layer or cell assembly, or
   - at least one electrode on the surface of the upper wall and/or side wall of the upper compartment and to at least one electrode on the surface of the bottom wall and/or side wall of the lower compartment if the porous membrane facing the lower and/or compartment is covered by said cells, cell layer or cell assembly,
      and
- measuring the impedance or capacitance.

The device of the present invention can also be used for determining the impedance of cells or a cell layer. The cells may be positioned on a porous membrane, upper wall, bottom wall and/or one or more side walls.

According to a preferred embodiment of the present invention air is applied to the upper compartment comprising a cell layer on the porous membrane positioned to the upper compartment to remove substantially all culture medium present in said upper compartment creating an air-liquid interface and alternating current is applied at least two electrodes on said porous membrane.

According to another preferred embodiment of the present invention air is applied to the lower compartment comprising a cell layer on the porous membrane positioned to the upper membrane surface in the lower compartment to remove substantially all culture medium present in said lower compartment creating an air-liquid interface and alternating current is applied at least two electrodes on said porous membrane.

Advantage of these embodiments compared to conventional TEER analysis is that cell analysis can be performed in a humid gaseous environment in the absence of medium. Conventional TEER approaches can only be performed when medium is resupplied and the air-liquid interface is discontinued during cell analysis.

The air applied to one of the compartments of the device of the present invention allows to determine the influence of gases on the cells of a cell layer. Thus, the composition of the air applied to one of the two compartments can be varied by introducing other gases or by changing its composition.

According to a preferred embodiment of the present invention the impedance and/or cell resistance and/or capacitance at the air-liquid interface is measured without the presence of an electrolyte in the upper or lower compartment. This means that cell monitoring at an air-liquid interface can be tested without the need for resupplementation of culture medium, which is a manipulation of the culture condition during cell analysis

According to a further preferred embodiment of the present invention the air applied to the lower or upper compartment comprises 78% nitrogen, 21 % oxygen, optionally the air applied to the compartments may contain carbon dioxide at 5% and nitrogen environmental conditions.

The alternating current is preferably applied to the electrodes of the device of the present invention at a frequency of 5 Hz to 5 MHz, preferably of 10 Hz to 2 Mhz.

The porous membrane comprising at least one electrode on its surface is produced using a method as described below. This method allows to cover porous and flexible membranes with electrodes of different structures.

A method for producing a porous membrane may comprise an electrode on its surface comprising the steps of
a) providing a solid support,
b) optionally depositing a water-soluble synthetic polymer or a water-insoluble synthetic polymer on said solid support,
c) placing a porous membrane on the solid support according to step a) or step b),
d) optionally depositing a layer comprising or consisting of a polydimethylglutarimide based resist, preferably LOR3A or LOR3B, with a thickness of about 0.1 to 2 pm, preferably about 0.2 to 1.5 µm, more preferably about 0.4 to 1 pm, more preferably about 0.6 µm, on the solid support of step c) ,
e) depositing a photoresist on the solid support of step c) or d),
f) aligning a photomask on the solid support of step e),
g) exposing the solid support of step f) to ultraviolet radiation,
h) applying a developer to the solid support of step (TMAH based) g),
i) subjecting the solid support of step h) to plasma, preferably argon or oxygen plasma,
j) depositing an electrode material on the solid support of step h) or i),
k) lift-off by soaking solid support of step j) in N-Ethyl-2-Pyrrolidon/N-Methyl-2-Pyrrolidon (NEP/NMP), and
l) releasing the membrane from the solid support of step k) using water or an aqueous solution.

This method is particularly advantageous because it allows applying electrodes on porous, preferably flexible, membranes. In particular the final step of the method wherein water or an aqueous solution instead of on organic solvent like acetone is used to release the membrane from the solid support allows manufacturing electrodes on porous membranes. The use of water, preferably deionized water, and aqueous solutions prevents the deterioration of the porous membranes and the electrodes during their manufacturing process.

"Aqueous solution", as used in the method of the present invention, may be any solution comprising more than 90 wt%, preferably more than 95 wt%, more preferably more than 98 wt%, water.

The solid support used to manufacture the porous membrane of the invention can be of any material. However, it is particularly preferred that the solid support comprises or consists of a material selected from the group consisting of glass, silicon, silicon nitride, silicon dioxide, zirconium dioxide.

The water-soluble synthetic polymer may be selected from the group consisting of polyvinyl alcohol (PVA), poly acrylic acids (PAA) or dextran.

2 to 10 wt%, preferably 3 to 5 wt%, more preferably approximately 4 wt%, polyvinyl alcohol with a molecular weight of 5,000 to 30,000, preferably 13,000 to 23,000, in deionised H₂O may be deposited on the solid support, so that surface is covered, the height of the deposited layer is defined by spin coating with 800rpm for 30s, in optional step b).

The solid support may be baked after step b), d), e) and/or g) by applying temperatures up to 180°C for up to 300 s.

The water-insoluble synthetic polymer may be selected from the group consisting of poly(methyl methacrylate), polystyrene, cyclic olefins (topas COC, zeonor COP), thiol-enes or thiol-enes-epoxies is deposited directly on said solid support. The water insoluble polymer is comprised within an appropriate solvent in an amount of 0.1 to 30 %w/v, preferably 1 to 25 %w/v.

The optional layer may comprise a polydimethylglutarimide based resist, preferably LOR3A or LOR3B, is applied on the solid support using spin deposition, preferably spin deposition at 500 to 2000 rpm for 15 to 60 s.

The photoresist is preferably selected from the group consisting of positive, negative or image reversal resists (e.g.:AZ5214E - novolak resin and naphthoquinone diazide based).

The photoresist may be deposited on the solid support of step c) or d) with a thickness of about 1 to 2.5 pm, preferably about 1.2 to 2 pm, more preferably about 1.5 to 1.7 pm, more preferably about 1,62µm, preferably by spin deposition at 1,000 to 5,000 rpm, preferably at 2,000 to 4,000 rpm, more preferably at approx. 3,000 rpm, for preferably 15 to 60 s, preferably approx. 30 s.

The solid support of step f) may be exposed to ultraviolet radiation at a dose of 5 to 500 mJ/cm², preferably of 10 to 400 mJ/cm², more preferably of 15 to 300 mJ/cm², more preferably of approx. 20 to 250 mJ/cm².

The developer applied to the solid support of step g) may be selected from the group consisting of Tetramethylammonium hydroxide (TMAH) and TMAH based developers (e.g.: AZ726MIF - containing 2,38% TMAH).

The electrode material may be selected from the group consisting of silver, gold, platinum, chromium, aluminium zinc oxide (AZO), indium tin oxide (ITO), iridium platinum, black platinum, titanium nitride and carbon.

The electrode material may be applied on the solid support of step h) or i) by sputtering, thermal evaporation, e-beam evaporation, screen printing or inkjet printing of conductive metal- or carbon-containing inks.

A porous membrane may be obtainable by a method as described herein.

A device according to the present invention may comprise a porous membrane as disclosed herein.

The present invention is further illustrated in the following figures and examples without being restricted thereto.

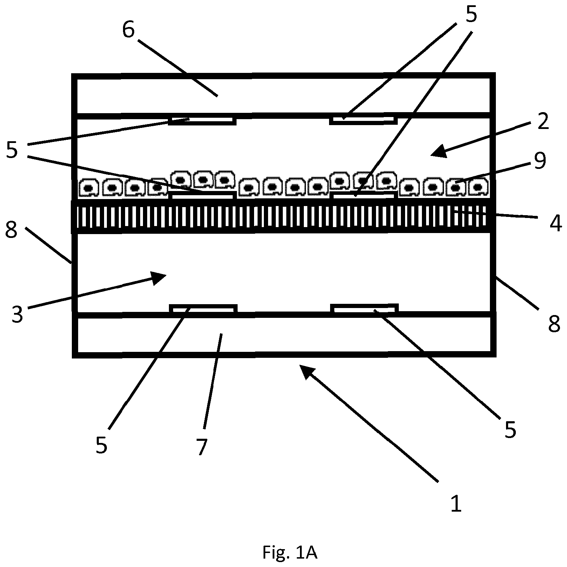

Fig. 1A shows a cross section of a microchannel 1 of a microfluidic device not within the scope of the present invention. Microchannel 1 comprises an upper compartment 2 and a lower compartment 3. Both compartments 2 and 3 are separated by a porous membrane 4. The lower compartment 3 comprises a bottom wall 7 and side walls 8. The upper compartment 2 comprises an upper wall 6 and side walls 8. The porous membrane 4 comprises on its surface at least one electrode 5. The upper wall 6 and the bottom wall 7 may comprise further electrodes 5. Porous membrane 4 and electrodes 5 on said membrane may be covered by a cell layer 9.

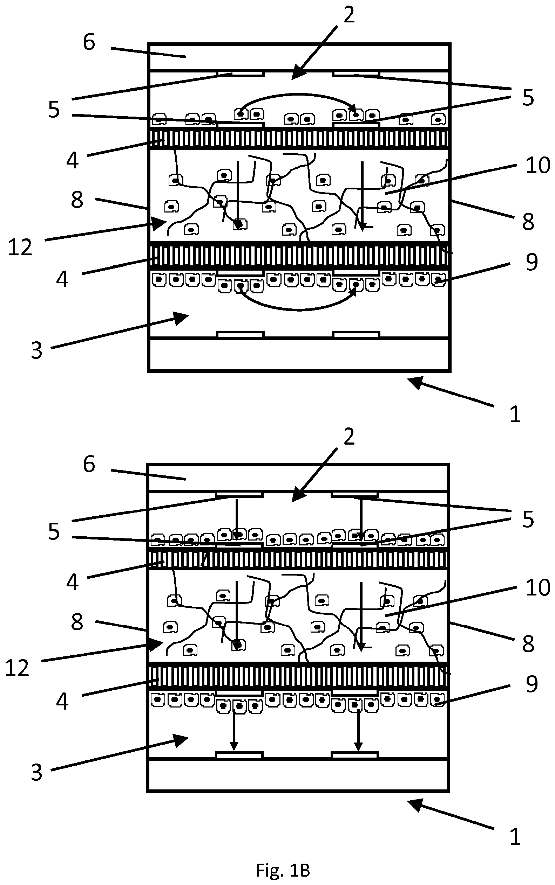

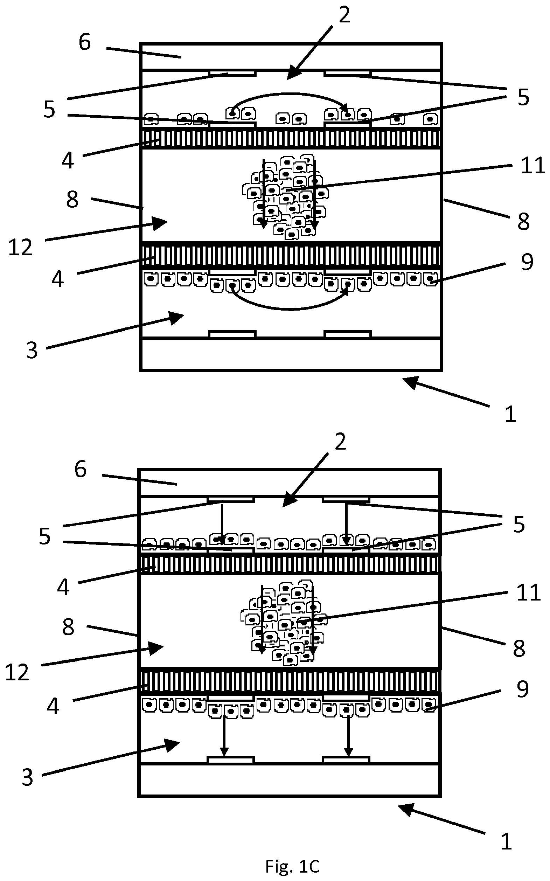

Figs. 1B and 1C show cross sections of a microchannel 1 comprising next to an upper compartment 2 and a lower compartment 3 an inner compartment 12, in accordance with the present invention. Said inner compartment 12 may comprise a cell assembly in form of a tissue 10 or a cell aggregate 11.

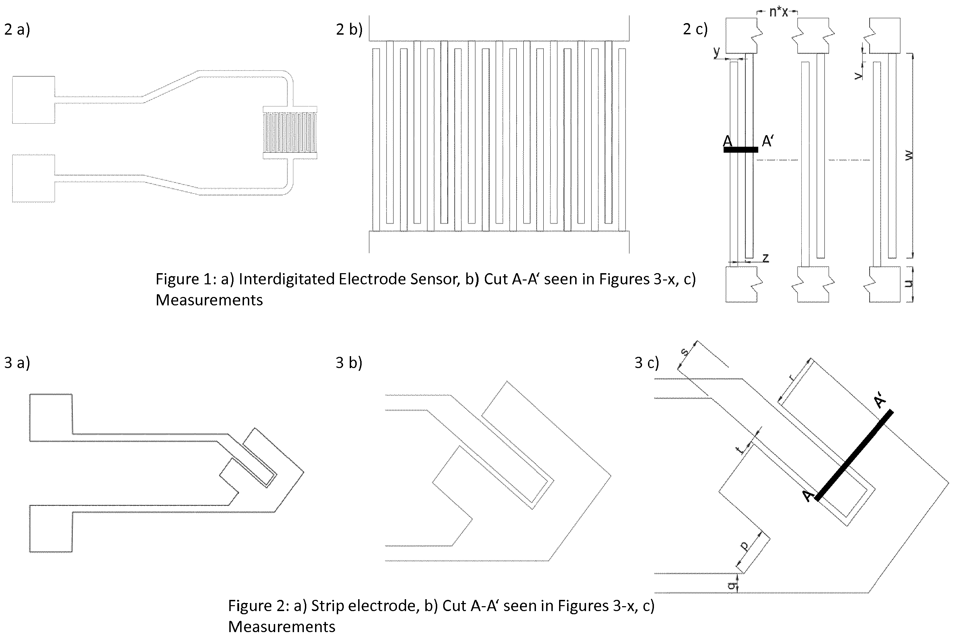

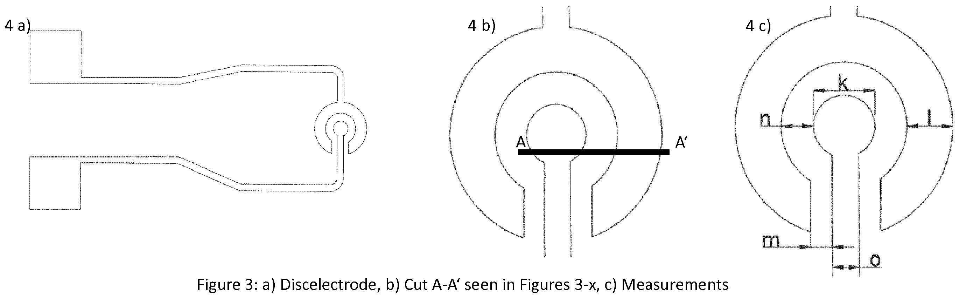

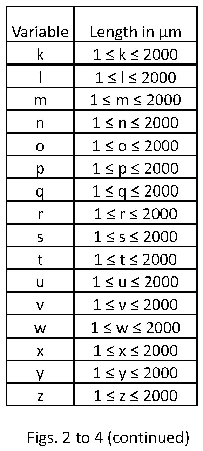

Figs. 2 to 4 show different electrode architectures which may be provided on the porous membrane, the upper wall, the lower wall and the side walls of the microchannel.

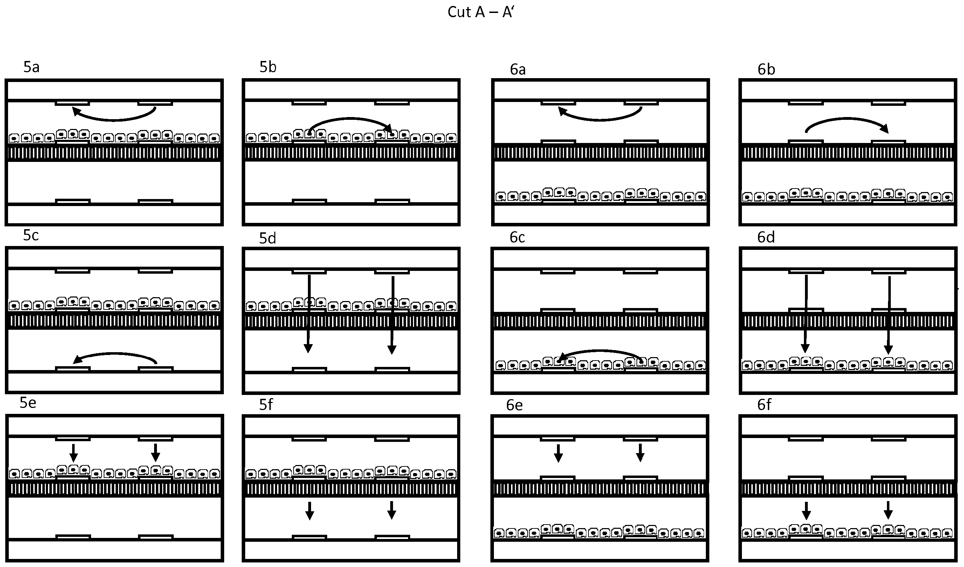

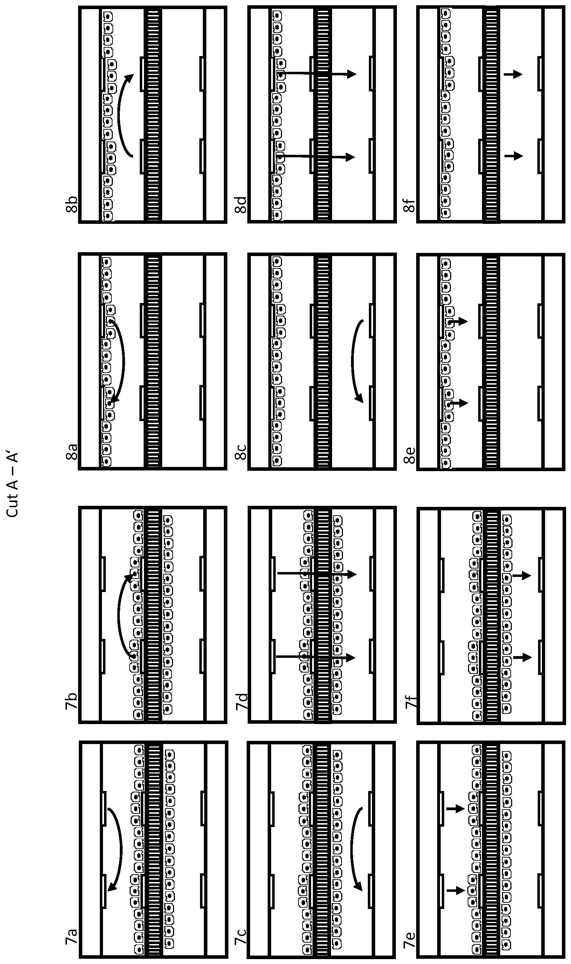

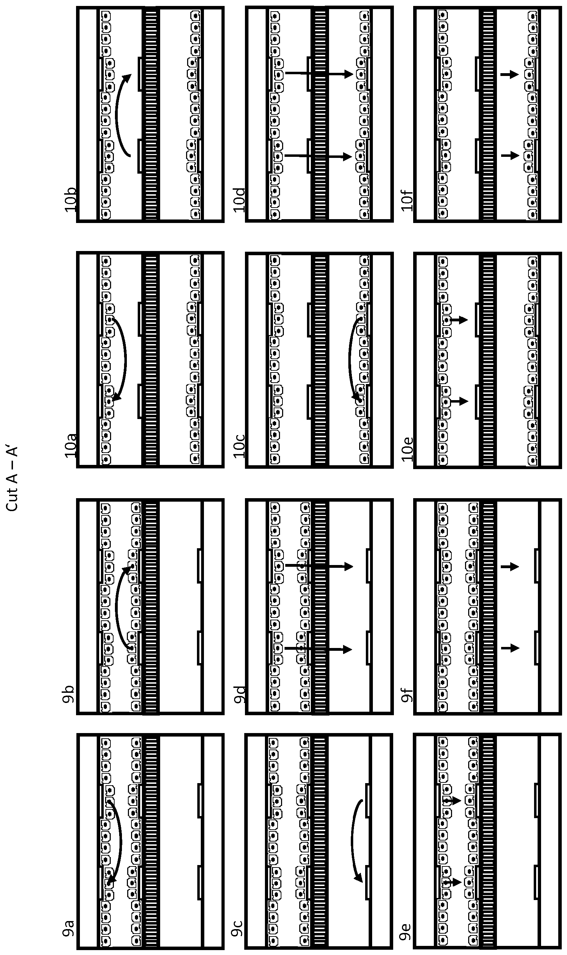

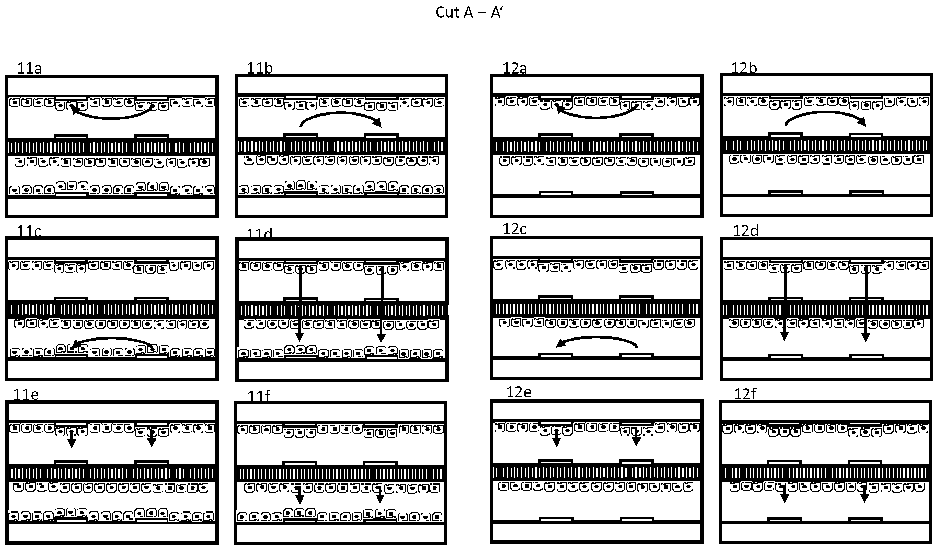

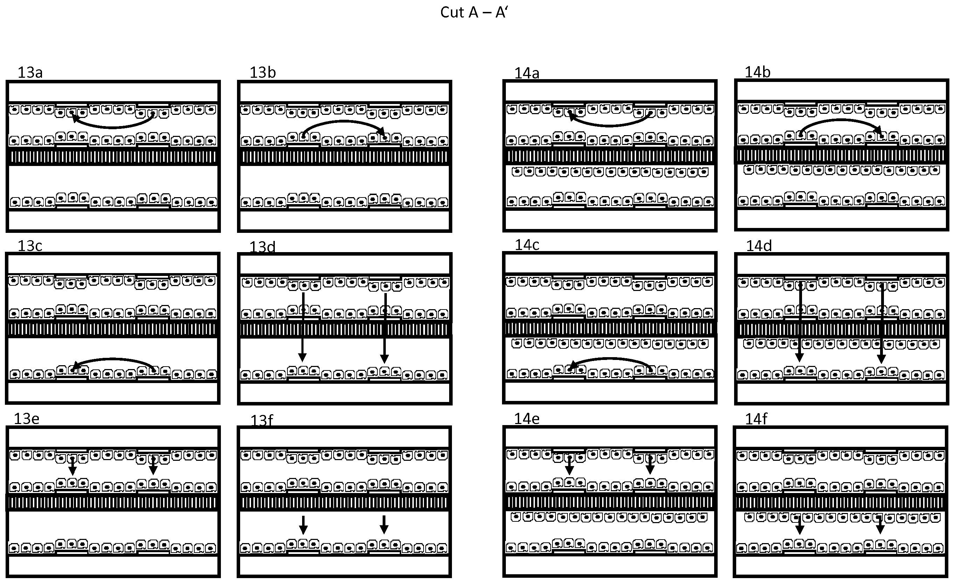

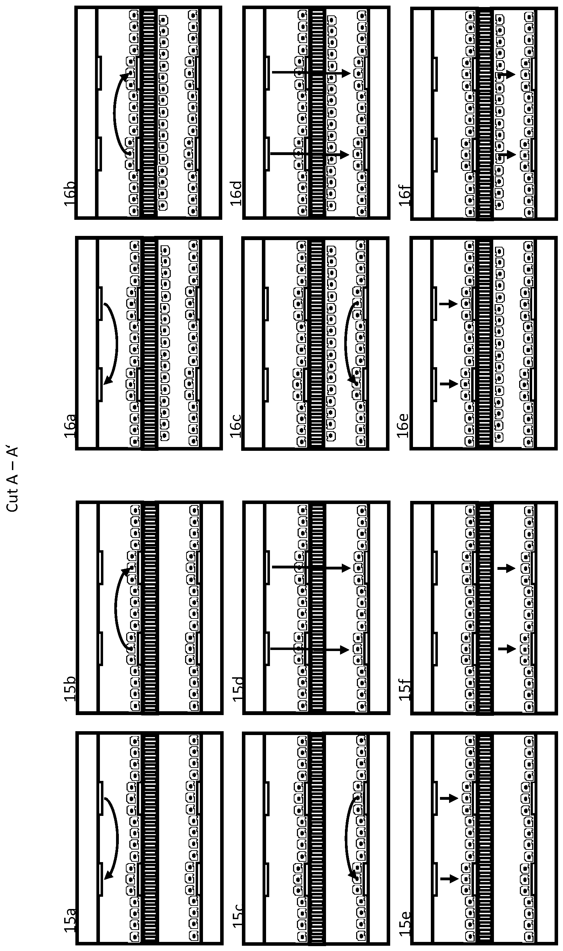

Figs. 5 to 16 are not within the scope of the present invention, showing cross sections of microchannels showing possible locations of the cell layers. The arrows indicate possible direction of the current flow.

### EXAMPLES

### Example 1: Fabrication of Membrane Electrodes

First the Polyvinyl alcohol (PVA) used as a release layer needs to be prepared. In order to allow for fast dissolution of the PVA only low molecular weight PVA (13000 - 23000 Da) should be used. 4g of PVA are dissolved in 100ml deionized H₂O (diH₂O) and stirred at 70°C (covered with aluminum foil to prevent evaporation of water) until the PVA is fully dissolved, once the PVA is dissolved the solution is filtered through a syringe filter (22um) to remove particles. Once the PVA is at room temperature it can be spin coated onto the carrier substrate (e.g. glass). The glass carrier substrate (Schott D263T eco) is cleaned using Acetone and Isopropyl alcohol and then placed on a hot plate set to 100°C to evaporate any remaining solvent. After cleaning the glass substrate is treated with O₂ plasma (300W; 0,7 Torr; 45 seconds) to allow for easier spreading of the PVA release layer. The plasma treated glass substrates are transferred to a spin coater and the PVA is spread using a transfer pipette or syringe and then spun at 800 rpm for 30 seconds. After spin coating of the PVA release layer, pre cut PET Membrane pieces (slightly larger than the carrier substrate) are carefully placed on the carrier substrate, trying to avoid wrinkles (it helps to bend the membrane a little) - once the membrane has been in contact with the PVA it shouldn't be moved anymore! The membrane should be placed onto the carrier while the PVA is still wet. In order to dry the PVA the substrates with the membrane attached are placed on a hotplate and temperature is ramped to 150°C (the LOR3A resist needs to be baked at this temperature). If no hotplate with a ramping function is available, or the ramping is too time consuming - the samples can be also baked gradually using hotplate set to 70°C, 100°C, 120° and 150°C for 180 seconds each. If the samples are baked too fast the evaporating water will cause wrinkles on the membrane. After dehydration the samples are cooled to room temperature, membrane pieces overlapping the carrier substrate are cut away using a scalpel and LOR3A resist is spin coated at 1000 rpm for 30s and then soft baked at 150°C for 180s - again the temperature should be ramped (or as mentioned above baked gradually). Once the LOR3A has been soft baked, AZ5214E Resist is spin coated at 3000 rpm for 30s and then soft baked at 100°C for 30s. Using a photo mask, the desired electrode geometry is transferred to the sample by UV light (365nm) exposure with a dose of 40 mJ/cm². After exposure the sample is baked at 120°C for 70s and then flood exposed (without photo mask) to a dose of 240 mJ/cm². Next the sample is developed in AZ726MIF (TMAH based developer) for 120s (usually AZ5214E needs to be developed for 60s - but TMAH dissolves LOR3A and allows for an undercut of the actual photo resist), and then rinsed in diH₂O. Before continuing the samples should be dried (e.g. with Nitrogen spray gun, overnight in a desiccator). Before depositing the metal layer, the samples are subjected to an Argon plasma (50W RF; 10sccm Ar; 60s), thereby modifying the parts of the membrane not covered by photoresist. After Plasma treatment a gold layer of approximately 80nm is deposited by sputtering (25W, 2x60s sputter duration, base pressure: 2e^-5 mbar, working pressure 8e^-3 mbar) or evaporation. The sputter power shouldn't exceed 25W, otherwise the membrane might overheat, or the metal might crack or spall during lift-off because of strain/tensile forces. After sputtering the samples are soaked in N-methyl pyrrolidone or N-Ethyl pyrrolidone for 10 minutes and the sonicated at low power to remove the photo resist and non-patterned gold. The membrane can be released by soaking the sample in diH₂O for 1 min and then carefully pulling it off with tweezers.

Using the process, gold electrodes can be deposited on porous membranes achieving a resolution down to 2.5 µm. This process can also be used to deposit other metals (e.g. copper, chromium, titanium), or combinations thereof. When depositing metal combinations using sputtering, only a low sputtering power should be used to avoid spalling or cracking of the metals. The PVA release layer allows for rapid detachment of the membrane from its carrier and aids further processing.

### Example 2: TEER and impedance measurements

### Material & Methods

Membrane electrodes were fabricated according to the Methods mentioned above, the microfluidic device was built by sand-blasting the culture chambers into microscope slides, and attached to the membrane using ARCare 90445 double sided adhesive tape. Microscope slides with drilled media inlet and outlet ports attached with ARCare 90445 were used to seal the chambers. As controls, BeWO b30 cells were seeded similarly on corning trans-well inserts with 3 µm pores and analyzed with a STX2 EVOM2 voltohmmeter after 45 minutes of cool-down.

Bewo B30 cells were routinely cultured in Ham's/F12 Media supplemented with 10% FBS, 1% Penicillin/Streptomycin and incubated at 37°C and 5%CO₂. Cells were detached from culture vessels using trypsin, centrifuged and seeded at different densities (100k/cm², 50k/cm², 25k/cm² and 12.5k/cm²) in culture media supplemented with 2% HEPES. Cells were propagated on the chip up to a duration of 5 days with daily medium exchange.

### Results & Discussion

Figure 17 A shows that the TEER measured with the proposed membrane-electrode setup in 2-point configuration yields comparable barrier integrity increase over time with respect to trans-well models tested with a STX2 EVOM2 voltohmmeter. As shown in Figure 17 B hen monitoring cell barriers in four-point configuration the proposed membrane electrode approach can even eliminate membrane resistance (n = 3) and can test purely cell-specific changes in resistance due to build-up or break-down of cell-to-cell junctions such as tight junctions. Figures 17 C and D show that membrane-based electrodes can be applied to investigate the evolution of cell barrier integrity (cellular electrical resistance due to tight junctions) using 100 µm finger-to-gap IDES (see Figure 17 C) and in parallel cell surface coverage using 15 µm finger-to-gap ratio (see Figure 17 D) with a multi-electrode approach for multi-parametric analysis of individual samples.

## Claims

1. A microfluidic device for determining the transepithelial electrical resistance (TEER) of a cell layer or a cell assembly and/or for determining the impedance of cells, a cell layer or a cell assembly, said device comprising at least one microchannel (1) comprising at least a lower (3) and an upper compartment (2) **characterised in that** said upper and lower compartments are separated by at least two porous membranes (4) and at least one inner compartment (12), the lower compartment (3) comprising a bottom wall (7) and side walls (8), the upper compartment (2) comprising an upper wall(6) and side walls (8), the bottom (7) and upper wall (6), the side walls (8) and the at least two porous membranes (4) defining compartment volumes, wherein at least one porous membrane (4) comprises on its surface at least one electrode (5), wherein the at least two porous membranes (4) and side walls define (8) the at least one inner compartment volume being positioned between the lower (3) and the upper (2) compartment.

2. The device according to claim 1, wherein at least one electrode (5) on at least one porous membrane (4) is facing the lower (3) and/or upper compartment (2) and/or at least one inner compartment (12).

3. The device according to claim 1 or 2, wherein the bottom (7) and/or upper wall (6) and/or at least one of the side walls (8) of one or more compartments comprises at least one electrode (5) on its surface.

4. The device according to any one of claims 1 to 3, wherein at least one porous membrane (4) and the bottom (7) and/or upper wall (6) and/or at least one of the side walls of one or more compartments comprise at least two electrodes (5) on their surface.

5. Method for determining the transepithelial electrical resistance (TEER) of a cell layer (9) or a cell assembly (10, 11) comprising the steps of
- providing a device according to any one of claims 1 to 5 comprising at least one electrode (5) on the surface of at least one porous membrane (4) facing the upper (2) and/or lower compartment (3) and/or an inner compartment (12) and at least one electrode (5) on the surface of the upper wall (6) and/or side wall (8) of the upper compartment (2) and/or the bottom wall (7) and/or side wall (8) of the lower compartment (3), wherein the porous membrane (4) is covered by a cell layer (9) or cell assembly (10),
- applying a direct current to
- at least one electrode (5) on said porous membrane (4) facing the upper compartment (2) or inner compartment (12) and to at least one electrode (5) on the surface of the upper wall (6) and/or side wall (8) of the upper compartment (2) if the porous membrane facing the upper compartment (2) is covered by a cell layer (9) or cell assembly (10), or
- at least one electrode (5) on said porous membrane (4) facing the lower compartment (3) or inner compartment (12) and to at least one electrode (5) on the surface of the upper wall (6) and/or side wall (8) of the upper compartment (2) if the porous membrane (4) facing the upper compartment (2) is covered by a cell layer (9) or cell assembly (10), or
- at least one electrode (5) on said porous membrane (4) facing the lower compartment (3) or inner compartment (12) and to at least one electrode (5) on the surface of the bottom wall and/or side wall of the lower compartment (3) if the porous membrane (4) facing the lower compartment (3) is covered by a cell layer (9) or cell assembly (10), or
- at least one electrode (5) on said porous membrane (4) facing the upper compartment (2) or inner compartment (12) and to at least one electrode (5) on the surface of the bottom wall (7) and/or side wall (8) of the lower compartment (3) if the porous membrane (4) facing the lower compartment (3) is covered by a cell layer (9) or cell assembly (10), or
- at least one electrode (5) on the surface of the upper wall (6) and/or side wall (8) of the upper compartment (2) and to at least one electrode (5) on the surface of the bottom wall (7) and/or side wall (8) of the lower compartment (3) if the porous membrane (4) facing the lower (3) and/or upper compartment (2) is covered by a cell layer (9) or cell assembly (10), or
- at least one electrode (5) on a first porous membrane (4) and to at least one electrode (5) on a second porous membrane (4), the first and the second porous membrane (4) defining the inner compartment (12), wherein the inner compartment (12) comprises a cell assembly (10) or a cell layer (9) covering the first and/or second porous membrane (4),
and
- measuring the electrical resistance.

6. Method for determining the impedance of cells, a cell layer (9) or cell assembly (10) comprising the steps of
- providing a device according to any one of claims 1 to 5 comprising at least one electrode (5) on the surface of at least one porous membrane (4) facing the upper (2) and/or lower compartment (3) and/or an inner compartment (12) and optionally at least one electrode (5) on the surface of the upper wall (6) and/or side wall (8) of the upper compartment (2) and/or the bottom wall (7) and/or side wall (8) of the lower compartment (3), wherein the porous membrane (4) is covered by cells, a cell layer (9) or a cell assembly (10),
- applying an alternating current to
- at least two electrodes on said porous membrane (4) being covered by said cells, cell layer (9) or cell assembly (10), or
- at least one electrode (5) on said porous membrane (4) facing the upper compartment (3) and to at least one electrode (5) on the surface of the upper wall (6) and/or side wall (8) of the upper compartment (2) if the porous membrane (4) facing the upper compartment (2) is covered by said cells, cell layer (9) or cell assembly (10), or
- at least one electrode (5) on said porous membrane (4) facing the lower compartment (3) and to at least one electrode (5) on the surface of the upper wall (6) and/or side wall (8) of the upper compartment (2) if the porous membrane (4) facing the upper compartment (2) is covered by said cells, cell layer (9) or cell assembly (10), or
- at least one electrode (5) on said porous membrane (4) facing the lower compartment (2) and to at least one electrode (5) on the surface of the bottom wall (7) and/or side wall (8) of the lower compartment (3) if the porous membrane (4) facing the lower compartment (3) is covered by said cells, cell layer (9) or cell assembly (10), or
- at least one electrode (5) on said porous membrane (4) facing the upper compartment (2) and to at least one electrode (5) on the surface of the bottom wall (7) and/or side wall (8) of the lower compartment (3) if the porous membrane (4) facing the lower compartment (3) is covered by said cells, cell layer (9) or cell assembly (10), or
- at least one electrode (5) on the surface of the upper wall (6) and/or side wall (8) of the upper compartment (2) and to at least one electrode (5) on the surface of the bottom wall (7) and/or side wall (8) of the lower compartment (3) if the porous membrane (4) facing the lower (3) and/or upper compartment (2) is covered by said cells, cell layer (9) or cell assembly (10),
and
- measuring the impedance or capacitance.

## Patentansprüche

1. Mikrofluidische Vorrichtung zur Bestimmung des transepithelialen elektrischen Widerstands (TEER) einer Zellschicht oder eines Zellverbands und/oder zur Bestimmung der Impedanz von Zellen, einer Zellschicht oder eines Zellverbands, wobei die Vorrichtung mindestens einen Mikrokanal (1) umfasst, der mindestens eine untere (3) und eine obere Kammer (2) umfasst, **dadurch gekennzeichnet, dass** die obere und die untere Kammer durch mindestens zwei poröse Membranen (4) und mindestens eine innere Kammer (12) getrennt sind, wobei die untere Kammer (3) eine Bodenwand (7) und Seitenwände (8) umfasst, wobei die obere Kammer (2) eine obere Wand (6) und Seitenwände (8) umfasst, wobei die Bodenwand (7) und die obere Wand (6), die Seitenwände (8) und die mindestens zwei porösen Membranen (4) Kammervolumina definieren, wobei mindestens eine poröse Membran (4) auf ihrer Oberfläche mindestens eine Elektrode (5) aufweist, wobei die mindestens zwei porösen Membranen (4) und die Seitenwände (8) das mindestens eine innere Kammervolumen definieren, das zwischen der unteren (3) und der oberen (2) Kammer positioniert ist.

2. Vorrichtung gemäß Anspruch 1, wobei mindestens eine Elektrode (5) auf mindestens einer porösen Membran (4) der unteren (3) und/oder oberen Kammer (2) und/oder mindestens einer inneren Kammer (12) zugewandt ist.

3. Vorrichtung gemäß Anspruch 1 oder 2, wobei die Bodenwand (7) und/oder die obere Wand (6) und/oder mindestens eine der Seitenwände (8) einer oder mehrerer Kammern mindestens eine Elektrode (5) auf ihrer Oberfläche aufweisen.

4. Vorrichtung gemäß einem der Ansprüche 1 bis 3, wobei mindestens eine poröse Membran (4) und die Bodenwand (7) und/oder die obere Wand (6) und/oder mindestens eine der Seitenwände einer oder mehrerer Kammern mindestens zwei Elektroden (5) auf ihrer Oberfläche aufweisen.

5. Verfahren zur Bestimmung des transepithelialen elektrischen Widerstands (TEER) einer Zellschicht (9) oder eines Zellverbands (10, 11), umfassend die folgenden Schritte:
- Bereitstellen einer Vorrichtung gemäß einem der Ansprüche 1 bis 5, umfassend mindestens eine Elektrode (5) auf der Oberfläche mindestens einer porösen Membran (4), die der oberen (2) und/oder unteren Kammer (3) und/oder einer inneren Kammer (12) zugewandt ist, und mindestens eine Elektrode (5) auf der Oberfläche der oberen Wand (6) und/oder Seitenwand (8) der oberen Kammer (2) und/oder der Bodenwand (7) und/oder der Seitenwand (8) der unteren Kammer (3), wobei die poröse Membran (4) von einer Zellschicht (9) oder einem Zellverband (10) bedeckt ist,
- Anlegen eines Gleichstroms
- an mindestens eine Elektrode (5) auf der porösen Membran (4), die der oberen Kammer (2) oder der inneren Kammer (12) zugewandt ist, und an mindestens eine Elektrode (5) auf der Oberfläche der oberen Wand (6) und/oder Seitenwand (8) der oberen Kammer (2), wenn die poröse Membran, die der oberen Kammer (2) zugewandt ist, von einer Zellschicht (9) oder einem Zellverband (10) bedeckt ist, oder
- an mindestens eine Elektrode (5) auf der porösen Membran (4), die der unteren Kammer (3) oder der inneren Kammer (12) zugewandt ist, und an mindestens eine Elektrode (5) auf der Oberfläche der oberen Wand (6) und/oder Seitenwand (8) der oberen Kammer (2), wenn die poröse Membran (4), die der oberen Kammer (2) zugewandt ist, von einer Zellschicht (9) oder einem Zellverband (10) bedeckt ist, oder
- an mindestens eine Elektrode (5) auf der porösen Membran (4), die der unteren Kammer (3) oder der inneren Kammer (12) zugewandt ist, und an mindestens eine Elektrode (5) auf der Oberfläche der Bodenwand und/oder Seitenwand der unteren Kammer (3), wenn die poröse Membran (4), die der unteren Kammer (3) zugewandt ist, von einer Zellschicht (9) oder einem Zellverband (10) bedeckt ist, oder
- an mindestens eine Elektrode (5) auf der porösen Membran (4), die der oberen Kammer (2) oder der inneren Kammer (12) zugewandt ist, und an mindestens eine Elektrode (5) auf der Oberfläche der Bodenwand (7) und/oder Seitenwand (8) der unteren Kammer (3), wenn die poröse Membran (4), die der unteren Kammer (3) zugewandt ist, von einer Zellschicht (9) oder einem Zellverband (10) bedeckt ist, oder
- an mindestens eine Elektrode (5) auf der Oberfläche der oberen Wand (6) und/oder Seitenwand (8) der oberen Kammer (2), und an mindestens eine Elektrode (5) auf der Oberfläche der Bodenwand (7) und/oder Seitenwand (8) der unteren Kammer (3), wenn die poröse Membran (4), die der unteren (3) und/oder oberen Kammer (2) zugewandt ist, von einer Zellschicht (9) oder einem Zellverband (10) bedeckt ist, oder
- an mindestens eine Elektrode (5) auf einer ersten porösen Membran (4) und an mindestens eine Elektrode (5) auf einer zweiten porösen Membran (4), wobei die erste und die zweite poröse Membran (4) die innere Kammer (12) definieren, wobei die innere Kammer (12) einen Zellverband (10) oder eine Zellschicht (9) umfasst, der bzw. die die erste und/oder zweite poröse Membran (4) bedeckt,
und
- Messen des elektrischen Widerstands.

6. Verfahren zur Bestimmung der Impedanz von Zellen, einer Zellschicht (9) oder eines Zellverbands (10), umfassend die folgenden Schritte:
- Bereitstellen einer Vorrichtung gemäß einem der Ansprüche 1 bis 5, umfassend mindestens eine Elektrode (5) auf der Oberfläche mindestens einer porösen Membran (4), die der oberen (2) und/oder unteren Kammer (3) und/oder einer inneren Kammer (12) zugewandt ist, und gegebenenfalls mindestens eine Elektrode (5) auf der Oberfläche der oberen Wand (6) und/oder Seitenwand (8) der oberen Kammer (2) und/oder der Bodenwand (7) und/oder Seitenwand (8) der unteren Kammer (3), wobei die poröse Membran (4) von Zellen, einer Zellschicht (9) oder einem Zellverband (10) bedeckt ist,
- Anlegen eines Wechselstroms
- an mindestens zwei Elektroden auf der porösen Membran (4), die von den Zellen, der Zellschicht (9) oder dem Zellverband (10) bedeckt ist, oder
- an mindestens eine Elektrode (5) auf der porösen Membran (4), die der oberen Kammer (3) zugewandt ist, und an mindestens eine Elektrode (5) auf der Oberfläche der oberen Wand (6) und/oder Seitenwand (8) der oberen Kammer (2), wenn die poröse Membran (4), die der oberen Kammer (2) zugewandt ist, von den Zellen, der Zellschicht (9) oder dem Zellverband (10) bedeckt ist, oder
- an mindestens eine Elektrode (5) auf der porösen Membran (4), die der unteren Kammer (3) zugewandt ist, und an mindestens eine Elektrode (5) auf der Oberfläche der oberen Wand (6) und/oder Seitenwand (8) der oberen Kammer (2), wenn die poröse Membran (4), die der oberen Kammer (2) zugewandt ist, von den Zellen, der Zellschicht (9) oder dem Zellverband (10) bedeckt ist, oder
- an mindestens eine Elektrode (5) auf der porösen Membran (4), die der unteren Kammer (2) zugewandt ist, und an mindestens eine Elektrode (5) auf der Oberfläche der Bodenwand (7) und/oder Seitenwand (8) der unteren Kammer (3), wenn die poröse Membran (4), die der unteren Kammer (3) zugewandt ist, von den Zellen, der Zellschicht (9) oder dem Zellverband (10) bedeckt ist, oder
- an mindestens eine Elektrode (5) auf der porösen Membran (4), die der oberen Kammer (2) zugewandt ist, und an mindestens eine Elektrode (5) auf der Oberfläche der Bodenwand (7) und/oder Seitenwand (8) der unteren Kammer (3), wenn die poröse Membran (4), die der unteren Kammer (3) zugewandt ist, von den Zellen, der Zellschicht (9) oder dem Zellverband (10) bedeckt ist, oder
- an mindestens eine Elektrode (5) auf der Oberfläche der oberen Wand (6) und/oder Seitenwand (8) der oberen Kammer (2), und an mindestens eine Elektrode (5) auf der Oberfläche der Bodenwand (7) und/oder Seitenwand (8) der unteren Kammer (3), wenn die poröse Membran (4), die der unteren (3) und/oder oberen Kammer (2) zugewandt ist, von den Zellen, der Zellschicht (9) oder dem Zellverband (10) bedeckt ist,
und
- Messen der Impedanz oder Kapazität.

## Revendications

1. Dispositif microfluidique pour déterminer la résistance électrique transépithéliale (TEER) d'une couche de cellules ou d'un ensemble de cellules et/ou pour déterminer l'impédance de cellules, d'une couche de cellules ou d'un ensemble de cellules, ledit dispositif comprenant au moins un microcanal (1) comprenant au moins un compartiment inférieur (3) et un compartiment supérieur (2), **caractérisé en ce que** lesdits compartiments supérieur et inférieur sont séparés par au moins deux membranes poreuses et au moins un compartiment intérieur (12), le compartiment inférieur (3) comprenant une paroi inférieure (7) et des parois latérales (8), le compartiment supérieur (2) comprenant une paroi supérieure (6) et des parois latérales (8), la paroi inférieure (7) et la paroi supérieure (6), les parois latérales (8) et les au moins deux membranes poreuses (4) définissant des volumes de compartiment, dans lequel au moins une membrane poreuse (4) comprend sur sa surface au moins une électrode (5), dans lequel les au moins deux membranes poreuses (4) et les parois latérales (8) définissent le au moins un volume de compartiment intérieur étant positionné entre le compartiment inférieur (3) et le compartiment supérieur (2).

2. Dispositif selon la revendication 1, dans lequel au moins une électrode (5) sur au moins une membrane poreuse (4) fait face au compartiment inférieur (3) et/ou au compartiment supérieur (2) et/ou au au moins un compartiment intérieur (12).

3. Dispositif selon la revendication 1 ou 2, dans lequel la paroi inférieure (7) et/ou la paroi supérieure (6) et/ou au moins une des parois latérales (8) d'un ou plusieurs compartiments comprennent au moins une électrode (5) sur leur surface.

4. Dispositif selon l'une quelconque des revendications 1 à 3, dans lequel au moins une membrane poreuse (4) et la paroi inférieure (7) et/ou la paroi supérieure (6) et/ou au moins une des parois latérales d'un ou plusieurs compartiments comprennent au moins deux électrodes (5) sur leur surface.

5. Procédé pour déterminer la résistance électrique transépithéliale (TEER) d'une couche de cellules (9) ou d'un ensemble de cellules (10, 11), comprenant les étapes consistant à
- fournir un dispositif selon l'une quelconque des revendications 1 à 5 comprenant au moins une électrode (5) sur la surface d'au moins une membrane poreuse (4) faisant face au compartiment supérieur (2) et/ou inférieur (3) et/ou un compartiment intérieur (12) et au moins une électrode (5) sur la surface de la paroi supérieure (6) et/ou de la paroi latérale (8) du compartiment supérieur (2) et/ou de la paroi inférieure (7) et/ou de la paroi latérale (8) du compartiment inférieur (3), dans lequel la membrane poreuse (4) est recouverte par une couche de cellules (9) ou un ensemble de cellule (10),
- appliquer un courant continu à
- au moins une électrode (5) sur ladite membrane poreuse (4) faisant face au compartiment supérieur (2) ou au compartiment intérieur (12) et à au moins une électrode (5) sur la surface de la paroi supérieure (6) et/ou de la paroi latérale (8) du compartiment supérieur (2) si la membrane poreuse faisant face au compartiment supérieur (2) est recouverte d'une couche de cellules (9) ou d'un ensemble de cellules (10), ou
- au moins une électrode (5) sur ladite membrane poreuse (4) faisant face au compartiment inférieur (3) ou au compartiment intérieur (12) et à au moins une électrode (5) sur la surface de la paroi supérieure (6) et/ou de la paroi latérale (8) du compartiment supérieur (2) si la membrane poreuse (4) faisant face au compartiment supérieur (2) est recouverte d'une couche de cellules (9) ou d'un ensemble de cellules (10), ou
- au moins une électrode (5) sur ladite membrane poreuse (4) faisant face au compartiment inférieur (3) ou au compartiment intérieur (12) et à au moins une électrode (5) sur la surface de la paroi inférieure et/ou de paroi latérale du compartiment inférieur (3) si la membrane poreuse (4) faisant face au compartiment inférieur (3) est recouverte d'une couche de cellules (9) ou d'un ensemble de cellules (10), ou
- au moins une électrode (5) sur ladite membrane poreuse (4) faisant face au compartiment supérieur (2) ou au compartiment intérieur (12) et à au moins une électrode (5) sur la surface de la paroi inférieure (7) et/ou de la paroi latérale (8) du compartiment inférieur (3) si la membrane poreuse (4) faisant face au compartiment inférieur (3) est recouverte d'une couche de cellules (9) ou d'un ensemble de cellules (10), ou
- au moins une électrode (5) sur la surface de la paroi supérieure (6) et/ou de la paroi latérale (8) du compartiment supérieur (2) et à au moins une électrode (5) sur la surface de la paroi inférieure (7) et/ou de la paroi latérale (8) du compartiment inférieur (3) si la membrane poreuse (4) faisant face au compartiment inférieur (3) et/ou supérieur (2) est recouverte d'une couche de cellules (9) ou d'un ensemble de cellules (10), ou
- au moins une électrode (5) sur une première membrane poreuse (4) et à au moins une électrode (5) sur une seconde membrane poreuse (4), les première et seconde membranes poreuses (4) définissant le compartiment intérieur (12), dans lequel le compartiment intérieur (12) comprend un ensemble de cellules (10) ou une couche de cellules (9) recouvrant la première et/ou la seconde membrane poreuse (4), et mesurant la résistance électrique.

6. Procédé pour déterminer l'impédance de cellules, d'une couche de cellules (9) ou d'un ensemble de cellules (10), comprenant les étapes consistant à
- fournir un dispositif selon l'une quelconque des revendications 1 à 5 comprenant au moins une électrode (5) sur la surface d'au moins une membrane poreuse (4) faisant face au compartiment supérieur (2) et/ou inférieur (3) et/ou un compartiment intérieur (12) et facultativement au moins une électrode (5) sur la surface de la paroi supérieure (6) et/ou de la paroi latérale (8) du compartiment supérieur (2) et/ou de la paroi inférieure (7) et/ou de la paroi latérale (8) du compartiment inférieur (3), dans lequel la membrane poreuse (4) est recouverte par des cellules, une couche de cellules (9) ou un ensemble de cellule (10),
- appliquer un courant alternatif à
- au moins deux électrodes sur ladite membrane poreuse (4) étant recouvertes par lesdites cellules, ladite couche de cellules (9) ou ledit ensemble de cellules (10), ou
- au moins une électrode (5) sur ladite membrane poreuse (4) faisant face au compartiment supérieur (3) et à au moins une électrode (5) sur la surface de la paroi supérieure (6) et/ou de la paroi latérale (8) du compartiment supérieur (2) si la membrane poreuse (4) faisant face au compartiment supérieur (2) est recouverte d'une couche de cellules (9) ou d'un ensemble de cellules (10), ou
- au moins une électrode (5) sur ladite membrane poreuse (4) faisant face au compartiment inférieur (3) et à au moins une électrode (5) sur la surface de la paroi supérieure (6) et/ou de la paroi latérale (8) du compartiment supérieur (2) si la membrane poreuse (4) faisant face au compartiment supérieur (2) est recouverte par lesdites cellules, ladit couche de cellules (9) ou ledit ensemble de cellules (10), ou
- au moins une électrode (5) sur ladite membrane poreuse (4) faisant face au compartiment inférieur (2) et à au moins une électrode (5) sur la surface de la paroi inférieure (7) et/ou de la paroi latérale (8) du compartiment inférieur (3) si la membrane poreuse (4) faisant face au compartiment inférieur (3) est recouverte par lesdites cellules, ladite couche de cellules (9) ou ledit ensemble de cellules (10), ou
- au moins une électrode (5) sur ladite membrane poreuse (4) faisant face au compartiment supérieur (2) et à au moins une électrode (5) sur la surface de la paroi inférieure (7) et/ou de la paroi latérale (8) du compartiment inférieur (3) si la membrane poreuse (4) faisant face au compartiment inférieur (3) est recouverte par lesdites cellules, ladite couche de cellules (9) ou ledit ensemble de cellules (10), ou
- au moins une électrode (5) sur la surface de la paroi supérieure (6) et/ou de la paroi latérale (8) du compartiment supérieur (2) et à au moins une électrode (5) sur la surface de la paroi inférieure (7) et/ou de la paroi latérale (8) du compartiment inférieur (3) si la membrane poreuse (4) faisant face au compartiment inférieur (3) et/ou supérieur (2) est recouverte par lesdites cellules, ladite couche de cellules (9) ou ledit ensemble de cellules (10), et
- mesurer l'impédance ou la capacité.
